## Europäisches Patentamt

## European Patent Office

## Office européen des brevets

(19)

(11) Veröffentlichungsnummer: **0 124 710**

**B1**

(12)

# EUROPÄISCHE PATENTSCHRIFT

(45) Veröffentlichungstag der Patentschrift:
24.09.86

(51) Int. Cl.⁴: **C 07 D 251/32**

(21) Anmeldenummer: **84102401.1**

(22) Anmeldetag: **07.03.84**

(54) **Verfahren zur Herstellung von reiner Cyanursäure.**

(30) Priorität: **09.05.83  DE 3316963**

(43) Veröffentlichungstag der Anmeldung:
**14.11.84 Patentblatt 84/46**

(45) Bekanntmachung des Hinweises auf die Patenterteilung:
**24.09.86 Patentblatt 86/39**

(84) Benannte Vertragsstaaten:
**AT BE CH DE FR GB IT LI NL**

(56) Entgegenhaltungen:
**EP-A-0 023 556**
**AT-B-365 180**
**CH-A-484 166**
**DE-A-1 067 027**
**US-A-2 943 088**
**US-A-3 325 493**

(73) Patentinhaber: **CHEMIE LINZ AKTIENGESELLSCHAFT, St. Peter- Strasse 25, A-4020 Linz (AT)**

(84) Benannte Vertragsstaaten: **BE CH FR GB IT LI NL AT**

(73) Patentinhaber: **Lentia Gesellschaft mit beschränkter Haftung, Arabellastrasse 4 Postfach 81 05 08, D-8000 München 81 (DE)**

(84) Benannte Vertragsstaaten: **DE**

(72) Erfinder: **Lunzer, Friedrich, Dipl.- Ing. Dr., Hostauerstrasse 27, A-4100 Ottensheim (AT)**
Erfinder: **Garber, Alfred, Dipl.- Ing., Ehrensteinweg 30, A-4020 Linz (AT)**

## Beschreibung

Die vorliegende Erfindung betrifft ein Verfahren zur Herstellung von reiner Cyanursäure durch Hydrolyse von Melamin, insbesondere von Roh- oder Abfallmelamin, das u. a. durch Ammelin oder Ammelid verunreinigt sein kann, mittels Schwefelsäure bei erhöhter Temperatur und unter erhöhtem Druck.

In der DE-AS 1,067.027 ist ein Verfahren zur Hydrolyse von Melamin, Ammelid oder Ammelin mit mindestens 5 %iger, vorzugsweise 10 - 20 %iger Schwefelsäure in geschlossenem Gefäß bei Temperaturen über 175° C und dem sich ergebenden Druck beschrieben. Die Bemessung der Schwefelsäuremenge soll laut Patentanspruch so erfolgen, daß gegenüber der stöchiometrischen Menge (3/2 Mol pro Mol Melamin) ein Überschuß von etwa 5 % zugegen ist. In jenen Beispielen, die die Hydrolyse von Melamin betreffen, wurden jedoch wesentlich größere Schwefelsäuremengen, nämlich 1,3 und mehr Mol Schwefelsäure pro Aminogruppenäquivalent im Melamin eingesetzt.

Die hohe Schwefelsäuremenge, der hohe sich ergebende Druck und das durch die große Verdünnung erforderliche große Volumen an teurem Druckreaktionsraum, machen dieses Verfahren technisch schwierig durchführbar und wenig wirtschaftlich.

In der AT-PS 365.180 wird ein bei Normaldruck arbeitendes, technisch sehr wohl durchführbares Verfahren zur Hydrolyse von Melamin mittels Schwefelsäure beschrieben, gemäß dem Melamin bei Raumtemperatur in 70 - 80 %ige Schwefelsäure eingetragen und anschließend unter Abdestillieren von Wasser das Gemisch innerhalb von 1 bis 2 Stunden auf 150 - 190° C aufgeheizt und dann zur Reaktion je nach gewählter Reaktionstemperatur 1,5 bis 6 Stunden auf dieser Temperatur gehalten wird, wobei mehrmals in das Reaktionsgemisch Wasser nachgegeben wird. Die bei diesem Verfahren angewendete Schwefelsäuremenge beträgt etwa 1 Mol pro Aminogruppenäquivalent, also 3 Mol pro Mol Melamin.

Dieses Verfahren hat den Nachteil, daß es nur diskontinuierlich durchgeführt werden kann, eine relativ lange Reaktionszeit benötigt und damit nur eine geringe Raumzeitausbeute erlaubt. Ferner hat sich in der Praxis gezeigt, daß die Reaktion sorgfältig überwacht werden muß, da sie zum Durchgehen unter teilweiser Vernichtung des Reaktionsansatzes neigt.

Es konnte nun überraschenderweise ein unter Druck arbeitendes Verfahren zur Hydrolyse von Melamin mittels Schwefelsäure gefunden werden, das die geschilderten Nachteile nicht zeigt, und bei guter Raum-Zeitausbeute und wesentlich kürzeren Reaktionszeiten zu reiner Cyanursäure führt. Dieses Verfahren, das auch kontinuierlich durchgeführt werden kann, macht sich die Erkenntnis zu nutze, daß Melamin dann mit konzentrierter bis mäßig verdünnter Schwefelsäure ohne Schwierigkeiten umgesetzt werden kann, wenn das Melamin in Form einer wäßrigen Suspension mit der Schwefelsäure gemischt wird, wobei die durch die Verdünnung entstehende Wärme zum Anspringen der Reaktion ausgenützt werden kann und wenn sowohl hinsichtlich der in das Gemisch eingebrachten Wassermenge als auch der Reaktionstemperatur gewisse Grenzen eingehalten werden.

Gegenstand der vorliegenden Erfindung ist demnach ein Verfahren zur Herstellung reiner Cyanursäure durch Hydrolyse von Melamin, insbesondere Roh- und Abfallmelamin, das gegebenenfalls Ammelin und Ammelid enthalten kann, bei einer Reaktionstemperatur von 160 - 200⁰C und erhöhtem Druck mittels etwa 3 Mol Schwefelsäure pro Mol Melamin, Zusatz von Wasser unter Abkühlen des Reaktionsgemisches auf Raumtemperatur und Isolierung, sowie Reinigung des in fester Form im Reaktionsgemisch anfallenden Produktes, das dadurch gekennzeichnet ist, daß das Melamin in Form einer wäßrigen Suspension mit mindestens 55%iger Schwefelsäure gemischt wird, wobei die mit der Schwefelsäure und der Melaminsuspension in das Reaktionssystem eingebrachte Wassermenge so bemessen wird, daß auf 1 Gewichtsteil Melamin mehr als 1 und bis zu 3,5 Gewichtsteile Wasser kommen, die Reaktion bei einem mindestens 2 bar betragenden, jedoch unterhalb des Reaktionsdruckes liegenden Druck durchgeführt und die Reaktionstemperatur von 160 -200° C durch temperatur- und/oder druckgeregeltes oder kontinuierliches Verdampfen eines Teiles des Wassers eingeregelt wird.

Das Abdestillieren des Wassers, das als Mittel zur Regulierung der Temperatur eingesetzt wird, kann auf jede beliebige Weise beispielsweise über einen regelbaren Rückflußkühler erfolgen. Bevorzugt wird hierzu jedoch ein auf Druckerhöhung ansprechendes Regelorgan, beispielsweise ein Entspannungsventil eingesetzt. Dadurch ist es auch möglich, unerwartete mit Druckanstieg verbundene Temperaturerhöhungen, die bei der exothermen Reaktion auftreten können, abzufangen. Für die vollständige Umsetzung in relativ kurzer Zeit, z.B. etwa 1/2 bis 1 Stunde, reicht ein Druck von 2 bis 10 bar völlig aus, sodaß die Reaktion vorteilhafterweise in üblichen Emailapparaturen durchgeführt werden kann, und damit keine gesonderten, teuren Apparaturen, die für höheren Druck ausgestattet sind, erforderlich sind.

Die Menge des Wassers, die mit dem Melamin eingetragen wird, hilft außerdem, die Reaktion zu bremsen. Je geringer die Wassermenge pro Teil Melamin ist, desto eher treten spontane Temperaturerhöhungen auf. Andererseits bringen zu große Wassermengen Nachteile mit sich und leidet die Raum-Zeitausbeute. Der Einsatz von 1,5 bis 2 Gew.Teilen Wasser pro Gew.Teil Melamin, hat sich dabei besonders bewährt, wobei damit die gesamte Wassermenge gemeint ist, die

sowohl mit der Säure, als auch mit der Melaminsuspension in das Reaktionsgemisch gelangt. Die Schwefelsäure kommt hierbei etwa in für die Ammonbisulfatbildung stöchiometrischer Menge zur Anwendung. Bevorzugt ist die Anwendung eines leichten Überschusses bis zu 1,2 Mol Schwefelsäure pro Aminogruppenäquivalent, wobei 1,05 bis 1,1 Mol Schwefelsäure pro Mol Aminogruppe besonders bevorzugt sind.

Das erfindungsgemäße Verfahren bietet die Möglichkeit, die Reaktion ohne jegliche Engergiezufuhr von außen durchzuführen, wenn bestimmte Verfahrensmaßnahmen eingehalten werden. Dazu gehört, daß die zur Umsetzung verwendete Schwefelsäure in nicht allzu geringer Konzentration innerhalb der erfindungsgemäßen Grenzen eingesetzt wird. Vorzugsweise wird von einer Schwefelsäure einer Konzentration von 76 bis 96% ausgegangen.

Bei diskontinuierlicher Fahrweise empfiehlt es sich ferner, die Schwefelsäure, die vorgelegt wird vorzuwärmen. Dies kann vorzugsweise dadurch geschehen, daß konzentrierte, vorzugsweise 96%ige Schwefelsäure vorgelegt wird und diese vor Zugabe der Melaminsuspension im Reaktionsgefäß auf die zur Mischung mit der Melaminsuspension gewünschte Konzentration verdünnt wird, wobei die dabei entstehende Wärme zur Vorwärmung ausgenützt wird. In diese so vorgewärmte Säure wird dann die Melaminsuspension eingetragen, wobei dabei ebenfalls freiwerdende Wärme die Einstellung der Reaktionstemperatur ohne Wärmezufuhr von außen ermöglicht.

Bei halbkontinuierlicher oder kontinuierlicher Fahrweise werden die Schwefelsäure und die Melaminsuspension gleichzeitig in das auf Reaktionstemperatur befindliche Reaktionsgemisch eingetragen. Auch hier ist in der Regel eine Wärmezufuhr von außen nicht erforderlich, vor allem dann nicht, wenn die Wassermenge innerhalb des bevorzugten Bereiches von 1,5 bis 2 Gew.Teilen Wasser pro Gew.Teil Melamin bleibt.

Im Gegensatz zur AT-PS 365.180 wird die Schwefelsäure also nur deshalb in konzentrierter Form eingesetzt, um die bei der Verdünnung auftretende Wärmetönung zum Aufwärmen des Reaktionsgemisches ausnützen zu können. Die Umsetzung selbst hingegen erfolgt mit einer Schwefelsäure geringerer Konzentration als in der AT-PS 365.180 vorgeschrieben, wobei deren Konzentration von der pro Gewichtsteil Melamin insgesamt eingebrachten Wassermenge bestimmt wird. Dabei ergibt sich auf Grund des Molverhältnisses von Melamin zu Schwefelsäure bei einem Gewichtsverhältnis von Wasser: Melamin von 1,25 : 1 etwa eine 67%ige Schwefelsäure, bei einem solchen von 2 : 1 etwa eine 55%ige Schwefelsäure, während bei Einsatz einer Wassermenge, die nahe der oberen Grenze, nämlich bei 3,35 : 1 liegt, der Umsatz mit etwa 40%iger Schwefelsäure vorgenommen wird. Der bevorzugte Bereich von Wasser zu Melamin von

1,5 bis 2 : 1 entspricht etwa einer Konzentration der tatsächlich zur Umsetzung kommenden Schwefelsäure von etwa 63 - 55%. Umso überraschender ist es, daß trotz der geringeren Konzentration der Schwefelsäure für die Umsetzung nur kürzere Reaktionszeiten benötigt werden.

Die Aufarbeitung erfolgt auf übliche Weise durch Abkühlen, Verdünnen mit Wasser und Abtrennen des kristallisierten Produktes durch Filtration oder Zentrifugieren. Nach Waschen des Produktes mit Klasser und Trocknen ist es bereits so rein, daß es keiner weiteren Reinigung mehr bedarf. Die Abkühlung erfolgt dabei wie üblich mit Kühlwasser, wobei zweckmäßig eine Temperatur unter 30° C erreicht werden sollte, da sonst die Ausbeute geringer wird.

Mit dem erfindungsgemäßen Verfahren kann in kurzer Zeit eine reine Cyanursäure mit einem Gehalt an Ammelin von unter 0,3% und einem solchen an Ammelid von ebenfalls unter 0,3% erhalten werden.

Die Raum-Zeit-Ausbeute ist gegenüber dem Stand der Technik schon allein deshalb viel größer, weil die Aufheizperiode wegfällt, und die Ausgangsmaterialien praktisch momentan auf Reaktionstemperatur gebracht werden. Die eigentliche Reaktionszeit richtet sich nach der gewählten Reaktionstemperatur. Sie liegt zwischen etwa 80 Minuten bei 160° C und etwa 5 Minuten bei 200° C. In dieser kurzen Zeit wird ein praktisch vollständiger Umsatz erzielt. An das zu verarbeitende Melamin sind keine besonderen Anforderungen bezüglich Reinheit zu stellen. Im Gegenteil, das erfindungsgemäße Verfahren bietet eine gute Möglichkeit der Verwertung von rohem Melamin, insbesondere Abfallmelamin, das unter anderem auch erhebliche Mengen an Ammelin und/oder Ammelid enthalten kann.

Die folgenden Ausführungsbeispiele sollen die Durchführung des erfindungsgemäßen Verfahrens näher erläutern, ohne es darauf beschränken zu wollen.

**Beispiel 1:**

1.222kg konzentrierter Schwefelsäure (96%ig = 12,0 kMol) werden in einem für Druck ausgestatteten Emaillekessel der mit Entspannungsventil versehen ist vorgelegt. Nach Zugabe von 80 kg Wasser werden 1.032 kg einer Melaminsuspension bestehend aus 458 kg Melamin ( = 3,63 kMol) und 574 kg H$_2$O eingetragen, das entspricht einem Verhältnis Gew.Teilen Melamin zu Gew.Teilen Wasser von 1 : 1,5. Die Temperatur steigt während der Melaminzugabe rasch auf 180° C an und wird durch eine Regelung, die auf das Entspannungsventil wirkt, gehalten. Der Druck beträgt etwa 4 bar. Anschließend wird noch eine halbe Stunde gerührt, auf 120 C gekuhlt, mit Wasser verdunnt, weiter auf unter 30 C gekuhlt, zentrifugiert, gewaschen und getrocknet. Es werden 454 kg reine Cyanursäure erhalten. Sowohl der Ammelingehalt als auch jener an Ammelid liegen unter 0,3%.

**Beispiel 2:**

In einem Emaillekessel mit Entspannungsventil werden zu etwa 400 kg Reaktionsmischung vom vorhergehenden Ansatz, die eine Temperatur von 190°C besitzt, gleichzeitig unter Rühren 917 kg konzentrierte Schwefelsäure und 914 kg Melaminsuspension bestehend aus 344 kg Melamin und 570 kg Wasser eingetragen. Mit Hilfe des Entspannungsventils wird soviel Wasserdampf abgelassen, daß die Temperatur von 190° C gehalten wird. Der maximale Druck beträgt 6 bar. Nach beendeter Eintragung wird noch 15 Minuten weitergerührt und danach das Reaktionsgemisch bis auf einen Rückstand von etwa 400 kg in den nächsten Kessel zur weiteren analog Beispiel 1 durchgeführten Aufarbeitung abgelassen. In 10 analogen Ansätzen wurden durchschnittlich 338 kg Cyanursäure (96% Ausbeute) erhalten. Der Gehalt an Ammelin lag unter 0,3%, jener an Ammelid ebenfalls unter 0,3%.

**Beispiel 3:**

In einem mit Rührer und Entspannungsventil versehenen 50 l Emaillekessel werden pro Stunde 733 kg konzentrierte Schwefelsäure und 743 kg einer Melaminsuspension bestehend aus 275 kg Melamin und 468 kg Wasser gepumpt (Gewichtsverhältnis Melamin zu Wasser 1: 1,8) und unter Rühren auf 200° C gehalten, indem gerade soviel Wasserdampf durch das Entspannungsventil abgelassen wird, daß die Temperatur von 200° C gehalten wird. Der Druck beträgt dabei etwa 10 bar.

Kontinuierlich wird das im Emaillekessel gebildete Reaktionsgemisch durch einen anschließenden Emaillerohrreaktor von 0,08 m³ Volumen gefahren. Das Reaktionsgemisch wird am Ende des Rohrreaktors entspannt, mit Wasser verdünnt und unter 30°C abgekühlt. Die auskristallisierte Cyanursäure wird abzentrifugiert, gewaschen und getrocknet. Pro Stunde werden reine 270 kg Cyanursäure mit einem Ammelingehalt unter 0,3% und einem Ammelidgehalt unter 0,3% erhalten.

Beispiele 4—12

Analog zu Beispiel 1 (Beispiele 4 und 5) und Beispiel 2 ((Beispiele 6—12) wurden folgende Versuche durchgeführt:

| | Melaminsuspension | | Schwefelsäure | | Gewichtsverh. | Temp. | Druck | Zeit | Ausbeute |
| | kg Melamin | kg H₂O | kg 96 % ige + kg H₂O | | H₂O: Melamin | °C | bar | min | % d.Th. |
|---|---|---|---|---|---|---|---|---|---|
| 4 | 458 | 574 | 1222 | 100 | 1,6 | 160 | 2 | 90 | 96 |
| 5 | 458 | 574 | 1222 | 200 | 1,8 | 170 | 3 | 40 | 96 |
| 6 | 257 | 323 | 687 | 512 | 3,35 | 170 | 6 | 40 | 95 |
| 7 | 419 | 480 | 1117 | - | 1,25 | 200 | 4 | 5 | 96 |
| 8 | 419 | 480 | 1036 | - | 1,24 | 200 | 5 | 8 | 96 |
| 9 | 347 | 590 | 1010 | - | 1,82 | 190 | 6 | 8 | 95 |
| 10 | 345 | 433 | 881 | 232 | 2,0 | 180 | 6 | 20 | 96 |
| 11 | 375 | 573 | 975 | - | 1,63 | 180 | 5 | 20 | 97 |
| 12 | 400 | 502 | 1068 | - | 1,36 | 180 | 3 | 20 | 96 |

Die Reinheit betrug in allen Fällen unter 0,3 % Ammelin und unter 0,3 % Ammelid.

**Patentansprüche:**

1. Verfahren zur Herstellung reiner Cyanursäure durch Hydrolyse von Melamin, insbesondere Roh- und Abfallmelamin, das gegebenenfalls Ammelin und Ammelid enthalten kann, bei einer Reaktionstemperatur von 160-200° C und erhöhtem Druck mittels etwa 3 Mol Schwefelsäure pro Mol Melamin, Zusatz von Wasser unter Abkühlen des Reaktionsgemisches auf Raumtemperatur und Isolierung, sowie Reinigung des in fester Form im Reaktionsgemisch anfallenden Produktes, dadurch gekennzeichnet, daß das Melamin in Form einer wäßrigen Suspension mit mindestens 55 %iger Schwefelsäure gemischt wird, wobei die mit der Schwefelsäure und der Melaminsuspension in das Reaktionssystem eingebrachte Wassermenge so bemessen wird, daß auf 1 Gewichtsteil Melamin mehr als 1 und bis zu 3,5 Gewichtsteile Wasser kommen, die Reaktion bei einem mindestens 2 bar betragenden jedoch unterhalb des Reaktionsdruckes liegenden Druck durchgeführt und die Reaktionstemperatur durch temperatur- und/oder druckgeregeltes oder kontinuierliches Verdampfen eines Teiles des Wassers eingeregelt wird.

2. Verfahren nach Anspruch 1, dadurch gekennzeichnet, daß das Abdestillieren des Wassers über ein auf Druckerhöhung ansprechendes Regelorgan erfolgt.

3. Verfahren nach den Ansprüchen 1 und 2, dadurch gekennzeichnet, daß der Druck 2 bis 10 bar beträgt.

4. Verfahren nach den Ansprüchen 1 bis 3, dadurch gekennzeichnet, daß die zugefügte Schwefelsäure eine Konzentration von 76 bis 96 % besitzt.

5. Verfahren nach den Ansprüchen 1 bis 4, dadurch gekennzeichnet, daß pro Gewichtsteil Melamin 1,5 bis 2 Gewichtsteile Wasser ins Reaktionsgemisch eingebracht werden.

6. Verfahren nach den Ansprüchen 1 bis 5, dadurch gekennzeichnet, daß pro Aminogruppenäquivalent 1,05 bis 1,1 Mol Schwefelsäure eingesetzt werden.

7. Verfahren nach den Ansprüchen 1 bis 6, dadurch gekennzeichnet, daß bei chargenweiser Durchführung des Verfahrens die Melaminsuspension in vorgelegte Schwefelsäure eingetragen wird, die durch unmittelbar vorangegangene Verdünnung auf die für die Mischung mit der Melaminsuspension gewünschte Konzentration vorgewärmt wurde.

8. Verfahren nach den Ansprüchen 1 bis 6, dadurch gekennzeichnet, daß es halbkontinuierlich oder kontinuierlich durchgeführt wird und die Schwefelsäure und die Melaminsuspension gleichzeitig in das auf Reaktionstemperatur befindliche Reaktionsgemisch eingetragen werden.

**Claims**

1. Process for the preparation of pure cyanuric acid by the hydrolysis of melamine, in particular crude and waste melamine, which can, if appropriate, contain ammeline and ammelide, at a reaction temperature of 160 - 200° C and elevated pressure, by means of about 3 moles of sulfuric acid per mole of melamine, by adding water while the reaction mixture is cooled to room temperature, and by isolating and purifying the product obtained in a solid form in the reaction mixture, characterized in that the melamine is mixed in the form of an aqueous suspension with at least 55% strength sulfuric acid, the amount of water introduced into the reaction system with the sulfuric acid and the melamine suspension being so adjusted that there are more than 1 and up to 3.5 parts by weight of water to 1 part by weight of melamine, the reaction being carried out under a pressure which is at least 2 bar, but is below the reaction pressure, and the reaction temperature being controlled by temperature-controlled and/or pressure-controlled or continuous evaporation of part of the water.

2. Process according to claim 1, characterized in that the removal of the water by distillation is effected via a control organ which responds to pressure increase.

3. Process according to claims 1 and 2, characterized in that the pressure is 2 to 10 bar.

4. Process according to claims 1 to 3, characterized in that the sulfuric acid added has a concentration of 76 to 96%.

5. Process according to claims 1 to 4, characterized in that 1.5 to 2 parts by weight of water are introduced into the reaction mixture per part by weight of melamine.

6. Process according to claims 1 to 5, characterized in that 1.05 to 1.1 moles of sulfuric acid are employed per amino group equivalent.

7. Process according to claims 1 to 6, characterized in that, if the process is carried out batchwise, the melamine suspension is introduced into sulfuric acid which has been initially taken and which has been preheated by being diluted immediately beforehand to the concentration desired for mixing with the melamine suspension.

8. Process according to claims 1 to 6, characterized in that it is carried out semicontinuously or continuously, and the sulfuric acid and the melamine suspension are introduced simultaneously into the reaction mixture which is at the reaction temperature.

**Revendications**

1.- Procédé pour la production d'acide cyanurique pur par hydrolyse de mélamine, en particulier de mélamine brute et formant déchet, qui peut contenir éventuellement de l'amméline et de l'ammélide, à une température de réaction de 160 - 200°C et sous pression élevée, au moyen d'environ 3 moles d'acide sulfurique par mole de mélamine, avec addition d'eau et avec refroidissement du mélange réactionnel et isolement, ainsi que purification du produit obtenu sous forme solide dans le mélange réactionnel, caractérisé en ce qu'on mélange la mélamine sous forme d'une suspension aqueuse avec de l'acide sulfurique à au moins 55 %, la quantité d'eau introduite dans le système réactionnel avec l'acide sulfurique et la suspension de mélamine étant mesurée de façon telle que l'on obtienne, pour 1 partie en poids de mélamine, plus de 1 et jusqu'à 3,5 parties en poids d'eau, on effectue la réaction sous une pression d'au moins 2 bars mais toutefois inférieure à la pression réactionnelle et on règle la température de réaction par évaporation d'une partie de l'eau, réglée par la température et/ou la pression, ou continue.

2.- Procédé suivant la revendication 1, caractérisé en ce que l'élimination par distillation de l'eau a lieu par l'intermédiaire d'un organe de régulation réagissant à une élévation de pression.

3.- Procédé suivant les revendications 1 et 2, caractérisé en ce que la pression est comprise entre 2 et 10 bars.

4.- Procédé suivant les revendications 1 à 3, caractérisé en ce que l'acide sulfurique ajouté a une concentration de 76 à 96 %.

5.- Procédé suivant les revendications 1 à 4, caractérisé en ce qu'on introduit, par partie en poids de mélamine, de 1,5 à 2 parties en poids d'eau dans le mélangé réactionnel.

6.- Procédé suivant les revendications 1 à 5, caractérisé en ce qu'on utilise, par équivalent de groupe amino, de 1,05 à 1,1 mole d'acide sulfurique.

7.- Procédé suivant les revendications 1 à 6, caractérisé en ce que, lors d'une mise en oeuvre du procédé par charges successives, on introduit la suspension de mélamine dans l'acide sulfurique déjà présent, qui a été préchauffé par dilution immédiatement précédente à la concentration désirée pour le mélange avec la suspension de mélamine.

8.- Procédé suivant les revendications 1 à 6, caractérisé en ce qu'il est mis en oeuvre de façon semicontinue ou continue et en ce qu'on introduit l'acide sulfurique et la suspension de mélamine de façon simultanée dans le mélange réactionnel se trouvant à la température de réaction.